# EUROPEAN PATENT SPECIFICATION

(11) **EP 1 334 120 B1**
(45) Date of publication and mention of the grant of the patent: **11.04.2012**
(21) Application number: 01996547.4
(22) Date of filing: 16.11.2001
(51) Int. Cl.: C07K 14/33

(54) **MODIFIED CLOSTRIDIAL NEUROTOXINS WITH ALTERED BIOLOGICAL PERSISTENCE**
MODIFIZIERTES CLOSTRIDIUMTOXIN MIT GEÄNDERTER BIOLOGISCHER PERSISTENZ
NEUROTOXINES CLOSTRIDIALES MODIFIEES A PERSISTANCE BIOLOGIQUE ALTEREE

(30) Priority: 17.11.2000 US 249540 P
(43) Date of publication of application: 13.08.2003
(73) Proprietor: ALLERGAN, INC., Irvine CA 92612 (US)
(72) Inventor: STEWARD, Lance, E., Irvine, CA 92614 (US); SPANOYANNIS, Athena, Tustin, CA 92782 (US); LIN, Wei-Jen, Cerritos, CA 90703 (US); AOKI, Kei, Roger, Coto de Caza, CA 92679 (US)
(74) Representative: HOFFMANN EITLE
(86) International application number: PCT/US2001/044030
(87) International publication number: WO 2002/040506

(56) References cited:
- WO-A-02/08268
- WO-A-96/39166
- WO-A-98/07864
- US-A- 5 989 545
- RACIBORSKA DOROTA A ET AL: "Retention of cleaved synaptosome-associated protein of 25 kDa (SNAP-25) in neuromuscular junctions: A new hypothesis to explain persistence of botulinum A poisoning." CANADIAN JOURNAL OF PHYSIOLOGY AND PHARMACOLOGY, vol. 77, no. 9, 1999, pages 679-688, XP009002209 ISSN: 0008-4212 cited in the application
- KURAZONO H ET AL: "MINIMAL ESSENTIAL DOMAINS SPECIFYING TOXICITY OF THE LIGHT CHAINS OF TETANUS TOXIN AND BOTULINUM NEUROTOXIN TYPE A" JOURNAL OF BIOLOGICAL CHEMISTRY, AMERICAN SOCIETY OF BIOLOGICAL CHEMISTS, BALTIMORE, MD, US, vol. 267, no. 21, 25 July 1992 (1992-07-25), pages 14721-14729, XP002047910 ISSN: 0021-9258
- FERRER-MONTIEL ANTONIO V ET AL: "Tyrosine phosphorylation modulates the activity of clostridial neurotoxins." JOURNAL OF BIOLOGICAL CHEMISTRY, vol. 271, no. 31, 1996, pages 18322-18325, XP002223867 ISSN: 0021-9258
- DE PAIVA A. ET AL.: PNAS USA, vol. 96, March 1999 (1999-03), pages 3200-3205,
- KELLER J. E. ET AL.: FEBS LETTERS, vol. 456, 1999, pages 137-142,
- SLOOP R.R. ET AL.: NEUROLOGY, vol. 49, no. 1, 1997, pages 189-194,

## Description

### BACKGROUND OF THE INVENTION

The present invention relates to modified BoNT/A-and BoNT/E- neurotoxins which are useful to treat various disorders, including neuromuscular disorders, autonomic nervous system disorders and pain.

The clinical use of botulinum toxin serotype A (herein after "BoNT/A"), a serotype of Clostridial neurotoxin, represents one of the most dramatic role reversals in modern medicine: a potent biologic toxin transformed into a therapeutic agent. BoNT/A has become a versatile tool in the treatment of a wide variety of disorders and conditions characterized by muscle hyperactivity, autonomic nervous system hyperactivity and/or pain.

### Botulinum toxin

The anaerobic, gram positive bacterium Clostridium botulinum produces a potent polypeptide neurotoxin, botulinum toxin, which causes a neuroparalytic illness in humans and animals referred to as botulism. The spores of Clostridium botulinum are found in soil and can grow in improperly sterilized and sealed food containers of home based canneries, which are the cause of many of the cases of botulism. The effects of botulism typically appear 18 to 36 hours after eating the foodstuffs infected with a Clostridium botulinum culture or spores. The botulinum toxin can apparently pass unattenuated through the lining of the gut and attack peripheral motor neurons. Symptoms of botulinum toxin intoxication can progress from difficulty walking, swallowing, and speaking to paralysis of the respiratory muscles and death.

BoNT/A is the most lethal natural biological agent known to man. About 50 picograms of botulinum toxin (purified neurotoxin complex) serotype A is a LD₅₀ in mice. One unit (U) of botulinum toxin is defined as the LD₅₀ upon intraperitoneal injection into female Swiss Webster mice weighing 18-20 grams each. Seven immunologically distinct botulinum neurotoxins have been characterized, these being respectively botulinum neurotoxin serotypes A, B, C₁, D, E, F and G each of which is distinguished by neutralization with serotype-specific antibodies. The different serotypes of botulinum toxin vary in the animal species that they affect and in the severity and duration of the paralysis they evoke. For example, it has been determined that BoNT/A is 500 times more potent, as measured by the rate of paralysis produced in the rat, than is botulinum toxin serotype B (BoNT/B). Additionally, BoNT/B has been determined to be non-toxic in primates at a dose of 480 U/kg which is about 12 times the primate LD₅₀ for BoNT/A. Botulinum toxin apparently binds with high affinity to cholinergic motor neurons, is translocated into the neuron and blocks the release of acetylcholine.

Botulinum toxins have been used in clinical settings for the treatment of neuromuscular disorders characterized by hyperactive skeletal muscles. BoNT/A has been approved by the U.S. Food and Drug Administration for the treatment of blepharospasm, strabismus and hemifacial spasm. Non-serotype A botulinum toxin serotypes apparently have a lower potency and/or a shorter duration of activity as compared to BoNT/A. Clinical effects of peripheral intramuscular BoNT/A are usually seen within one week of injection. The typical duration of symptomatic relief from a single intramuscular injection of BoNT/A averages about three months.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum serotypes A and E both cleave the 25 kiloDalton (kD) synaptosomal associated protein (SNAP-25), but they target different amino acid sequences within this protein. BoNT/B, D, F and G act on vesicle-associate protein (VAMP, also called synaptobrevin), with each serotype cleaving the protein at a different site. Finally, botulinum toxin serotype C₁ (BoNT/C₁) has been shown to cleave both syntaxin and SNAP-25. These differences in mechanism of action may affect the relative potency and/or duration of action of the various botulinum toxin serotypes.

Regardless of serotype, the molecular mechanism of toxin intoxication appears to be similar and to involve at least three steps or stages. In the first step of the process, the toxin binds to the presynaptic membrane of the target neuron through a specific interaction between the H chain and a cell surface receptor; the receptor is thought to be different for each serotype of botulinum toxin and for tetanus toxin. The carboxyl end segment of the H chain, H_{c}. appears to be important for targeting of the toxin to the cell surface.

In the second step, the toxin crosses the plasma membrane of the poisoned cell. The toxin is first engulfed by the cell through receptor-mediated endocytosis, and an endosome containing the toxin is formed. The toxin then escapes the endosome into the cytoplasm of the cell. This last step is thought to be mediated by the amino end segment of the H chain, Hₙ, which triggers a conformational change of the toxin in response to a pH of about 5.5 or lower. Endosomes are known to possess a proton pump which decreases intra endosomal pH. The conformational shift exposes hydrophobic residues in the toxin, which permits the toxin to embed itself in the endosomal membrane. The toxin then translocates through the endosomal membrane into the cytosol.

The last step of the mechanism of botulinum toxin activity appears to involve reduction of the disulfide bond joining the H and L chain. The entire toxic activity of botulinum and tetanus toxins is contained in the L chain of the holotoxin; the L chain is a zinc (Zn++) endopeptidase which selectively cleaves proteins essential for recognition and docketing of neurotransmitter-containing vesicles with the cytoplasmic surface of the plasma membrane, and fusion of the vesicles with the plasma membrane. Tetanus neurotoxin, botulinum toxin/B/D,/F, and/G cause degradation of synaptobrevin (also called vesicle-associated membrane protein (VAMP)), a synaptosomal membrane protein. Most of the VAMP present at the cytosolic surface of the synaptic vesicle is removed as a result of any one of these cleavage events. Each toxin specifically cleaves a different bond.

The molecular weight of the botulinum toxin protein molecule, for all seven of the known botulinum toxin serotypes, is about 150 kD. Interestingly, the botulinum toxins are released by Clostridial bacterium as complexes comprising the 150 kD botulinum toxin protein molecule along with associated non-toxin proteins. Thus, the BoNT/A complex can be produced by Clostridial bacterium as 900 kD, 500 kD and 300 kD forms. BoNT/B and C₁ are apparently produced as only a 500 kD complex. BoNT/D is produced as both 300 kD and 500 kD complexes. Finally, BoNT/E and F are produced as only approximately 300 kD complexes. The complexes (i.e. molecular weight greater than about 150kD) are believed to contain a non-toxin hemaglutinin protein and a non-toxin and non-toxic nonhemaglutinin protein. These two non-toxin proteins (which along with the botulinum toxin molecule comprise the relevant neurotoxin complex) may act to provide stability against denaturation to the botulinum toxin molecule and protection against digestive acids when toxin is ingested. Additionally, it is possible that the larger (greater than about 150 kD molecular weight) botulinum toxin complexes may result in a slower rate of diffusion of the botulinum toxin away from a site of intramuscular injection of a botulinum toxin complex.

In vitro studies have indicated that botulinum toxin inhibits potassium cation induced release of both acetylcholine and norepinephrine from primary cell cultures of brainstem tissue. Additionally, it has been reported that botulinum toxin inhibits the evoked release of both glycine and glutamate in primary cultures of spinal cord neurons and that in brain synaptosome preparations botulinum toxin inhibits the release of each of the neurotransmitters acetylcholine, dopamine, norepinephrine, CGRP and glutamate.

BoNT/A can be obtained by establishing and growing cultures of Clostridium botulinum in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the BoNT/B serotype only cleave a portion of the toxin produced. The exact proportion of nicked to unnicked molecules depends on the length of incubation and the temperature of the culture. Therefore, a certain percentage of any preparation of, for example, the BoNT/B toxin is likely to be inactive, possibly accounting for the known significantly lower potency of BoNT/B as compared to BoNT/A. The presence of inactive botulinum toxin molecules in a clinical preparation will contribute to the overall protein load of the preparation, which has been linked to increased antigenicity, without contributing to its clinical efficacy. Additionally, it is known that BoNT/B has, upon intramuscular injection, a shorter duration of activity and is also less potent than BoNT/A at the same dose level.

It has been reported that BoNT/A has been used in clinical settings as follows:
(1) about 75-125 units of BOTO^{®1} per intramuscular injection (multiple muscles) to treat cervical dystonia;.
(2) 5-10 unites of BOTO^{®} per intramuscular injection to treat glabellar lines (brow furrows) (5 units injected intramuscularly into the procerus muscle and 10 units injected intramuscularly into each corrugator supercilii muscle);
   ¹ Available from Allergan, Inc., of Irvine, California under the tradename BOTOX®.
(3) about 30-80 units of BOTO^{®} to treat constipation by intrasphincter injection of the puborectalis muscle;
(4) about 1-5 units per muscle of intramuscularly injected BOTOX^{®} to treat blepharospasm by injecting the lateral pre-tarsal orbicularis oculi muscle of the upper lid and the lateral pre-tarsal orbicularis-oculi of the lower lid.
(5) to treat strabismus, extraocular muscles have been injected intramuscularly with between about 1-5 units of BOTOX^{®}, the amount injected varying based upon both the size of the muscle to be injected and the extent of muscle paralysis desired (i.e. amount of diopter correction desired).
(6) to treat upper limb spasticity following stroke by intramuscular injections of BOTOX^{®} into five different upper limb flexor muscles, as follows:
   (a) flexor digitorum profundus: 7.5 U to 30 U
   (b) flexor digitorum sublimes: 7.5 U to 30 U
   (c) flexor carpi ulnaris: 10 U to 40 U
   (d) flexor carpi radialis: 15 U to 60 U
   (e) biceps brachii: 50 U to 200 U. Each of the five indicated muscles has been injected at the same treatment session, so that the patient receives from 90 U to 360 U of upper limb flexor muscle BOTOX^{®} by intramuscular injection at each treatment session.

The success of BoNT/A to treat a variety of clinical conditions has led to interest in other botulinum toxin serotypes. A study of two commercially available BoNT/A preparations (BOTOX^{®} and Dysport^{®}) and preparations of BoNT/B and F (both obtained from Wako Chemicals, Japan) has been carried out to determine local muscle weakening efficacy, safety and antigenic potential. Botulinum toxin preparations were injected into the head of the right gastrocnemius muscle (0.5 to 200.0 units/kg) and muscle weakness was assessed using the mouse digit abduction scoring assay (DAS). ED₅₀ values were calculated from dose response curves. Additional mice were given intramuscular injections to determine LD₅₀ doses. The therapeutic index was calculated as LD₅₀/ED₅₀. Separate groups of mice received hind limb injections of BOTOX^{®} (5.0 to 10.0 units/kg) or BoNT/B (50.0 to 400.0 units/kg), and were tested for muscle weakness and increased water consumption, the later being a putative model for dry mouth. Antigenic potential was assessed by monthly intramuscular injections in rabbits (1.5 or 6.5 ng/kg for BoNT/B or 0.15 ng/kg for BOTO^{®}). Peak muscle weakness and duration were dose related for all serotypes. DAS ED₅₀ values (units/kg) were as follows: BOTOX^{®}: 6.7, Dysport^{®}: 24.7, BoNT/B: 27.0 to 244.0, BoNT/F: 4.3. BOTOX^{®} had a longer duration of action than BoNT/B or BoNT/F. Therapeutic index values were as follows: BOTOX^{®}: 10.5, Dysport^{®}: 6.3, BoNT/B: 3.2. Water consumption was greater in mice injected with BoNT/B than with BOTOX^{®}, although BoNT/B was less effective at weakening muscles. After four months of injections 2 of 4 (where treated with 1.5 ng/kg) and 4 of 4 (where treated with 6.5 ng/kg) rabbits developed antibodies against BoNT/B. In a separate study, 0 of 9 BOTOX^{®} treated rabbits demonstrated antibodies against BoNT/A. DAS results indicate relative peak potencies of BoNT/A being equal to BoNT/F, and BoNT/F being greater that BoNT/B. With regard to duration of effect, BoNT/A was greater than BoNT/B, and BoNT/B duration of effect was greater than BoNT/F. As shown by the therapeutic index values, the two commercial preparations of BoNT/A (BOTOX^{®} and Dysport^{®}) are different. The increased water consumption behavior observed following hind limb injection of BoNT/B indicates that clinically significant amounts of this serotype entered the murine systemic circulation. The results also indicate that in order to achieve efficacy comparable to BoNT/A, it is necessary to increase doses of the other serotypes examined. Increased dosage can comprise safety. Furthermore, in rabbits, serotype B was more antigenic than was BOTO^{®}, possibly because of the higher protein load injected to achieve an effective dose of BoNT/B.

The tetanus neurotoxin acts mainly in the central nervous system, while botulinum neurotoxin acts at the neuromuscular junction; both act by inhibiting acetylcholine release from the axon of the affected neuron into the synapse, resulting in paralysis. The effect of intoxication on the affected neuron is long lasting and until recently has been thought to be irreversible. The tetanus neurotoxin is known to exist in one immunologically distinct serotype.

### Acetylcholine

Typically only a single type of small molecule neurotransmitter is released by each type of neuron in the mammalian nervous system. The neurotransmitter acetylcholine is secreted by neurons in many areas of the brain, but specifically by the large pyramidal cells of the motor cortex, by several different neurons in the basal ganglia, by the motor neurons that innervate the skeletal muscles, by the preganglionic neurons of the autonomic nervous system (both sympathetic and parasympathetic), by the postganglionic neurons of the parasympathetic nervous system, and by some of the postganglionic neurons of the sympathetic nervous system. Essentially, only the postganglionic sympathetic nerve fibers to the sweat glands, the piloerector muscles and a few blood vessels are cholinergic and most of the postganglionic neurons of the sympathetic nervous system secret the neurotransmitter norepinephrine. In most instances acetylcholine has an excitatory effect. However, acetylcholine is known to have inhibitory effects at some of the peripheral parasympathetic nerve endings, such as inhibition of the heart by the vagal nerve.

The efferent signals of the autonomic nervous system are transmitted to the body through either the sympathetic nervous system or the parasympathetic nervous system. The preganglionic neurons of the sympathetic nervous system extend from preganglionic sympathetic neuron cell bodies located in the intermediolateral horn of the spinal cord. The preganglionic sympathetic nerve fibers, extending from the cell body, synapse with postganglionic neurons located in either a paravertebral sympathetic ganglion or in a prevertebral ganglion. Since, the preganglionic neurons of both the sympathetic and parasympathetic nervous system are cholinergic, application of acetylcholine to the ganglia will excite both sympathetic and parasympathetic postganglionic neurons.

Acetylcholine activates two types of receptors, muscarinic and nicotinic receptors. The muscarinic receptors are found in all effector cells stimulated by the postganglionic neurons of the parasympathetic nervous system, as well as in those stimulated by the postganglionic cholinergic neurons of the sympathetic nervous system. The nicotinic receptors are found in the synapses between the preganglionic and postganglionic neurons of both the sympathetic and parasympathetic. The nicotinic receptors are also present in many membranes of skeletal muscle fibers at the neuromuscular junction.

Acetylcholine is released from cholinergic neurons when small, clear, intracellular vesicles fuse with the presynaptic neuronal cell membrane. A wide variety of non-neuronal secretory cells, such as, adrenal medulla (as well as the PC12 cell line) and pancreatic islet cells release catecholamines and insulin, respectively, from large dense-core vesicles. The PC12 cell line is a clone of rat pheochromocytoma cells extensively used as a tissue culture model for studies of sympathoadrenal development. Botulinum toxin inhibits the release of both types of compounds from both types of cells in vitro, permeabilized (as by electroporation) or by direct injection of the toxin into the denervated cell. Botulinum toxin is also known to block release of the neurotransmitter glutamate from cortical synaptosomes cell culture.

Sanders et al. in U.S. Patent No. 5,766,605 (Sanders et al.) disclose that BoNT/A can be used to treat autonomic nervous system disorders, for example rhinorrhea, otitis media, excessive salivation, asthma, chronic obstructive pulmonary disease (COPD), excessive stomach acid secretion, spastic colitis and excessive sweating.

Furthermore, Binder in U.S.. Patent No. 5,714,468 (Binder) discloses that BoNT/A can be used to treat migraine headache pain that is associated with muscle spasm, vascular disturbances, neuralgia and neuropathy. Additionally, Kei et al. in U.S. Patent 6,113,915 (Kei et al.) disclose that BoNT, for example BoNT/A, may be used to treat pain, for example neuropathic or inflammatory pain.

One of the reasons that BoNT/A has been selected over the other serotypes, for example serotypes B, C₁, D, E, F and G, for clinical use is that BoNT/A has a substantially longer lasting therapeutic effect. In other words, the inhibitory effect of BoNT/A is more persistent. Therefore, the other serotypes of botulinum toxins could potentially be effectively used in a clinical environment if their biological persistence could be enhanced. For example, parotoid sialocele is a condition where the patient suffers from excessive salivation. Sanders et al. disclose in their patent that serotype D may be very effective in reducing excessive salivation. However, the biological persistence of serotype D botulinum toxin is relatively short and thus may not be practical for clinical use. If the biological persistence of serotype D may be enhanced, it may effectively be used in a clinical environment to treat, for example, parotid sialocele.

Another reason that BoNT/A has been a preferred neurotoxin for clinical use is, as discussed above, its superb ability to immobilize muscles through flaccid paralysis. For example, BoNT/A is preferentially used to immobilize muscles and prevent limb movements after a tendon surgery to facilitate recovery. However, for some minor tendon surgeries, the healing time is relatively short. It would be beneficial to have a BoNT/A without the prolonged persistence for use in such circumstances so that the patient can regain mobility at about the same time the recover from the surgery.

Presently, the basis for the differences in persistence among the various botulinum toxins is unknown. However, there are two main theories explaining the differences in the persistence of the toxins. Without wishing to be bound by any theory of operation or mechanism of action, these theories will be discussed briefly below. The first theory proposes that the persistence of a toxin depends on which target protein and where on that target protein that toxin attacks. Raciborska et al., Can. J. Physiol. Pharmcol. 77:679-688 (1999). For example, SNAP-25 and VAMP are proteins required for vesicular docking, a necessary step for vesicular exocytosis. BoNT/A cleaves the target protein SNAP-25 and BoNT/B cleaves the target protein VAMP, respectively. The effect of each is similar in that cleavage of either protein compromises the ability of a neuron to release neurotransmitters via exocytosis. However, damaged VAMP may be more easily replaced with new ones that damaged SNAP-25, for example by replacement synthesis. Therefore, since it takes longer for cells to synthesize new SNAP-25 proteins to replace damaged ones, BoNT/A has longer persistence. *Id*. At 685.

Additionally, the site of cleavage by a toxin may dictate how quickly the damaged target proteins may be replaced. For example, BoNT/A and E both cleave SNAP-25. However, they cleave at different sites and BoNT/E causes shorter-lasting paralysis in patients, compared with BoNT/A. *Id.* At 685-6.

The second theory proposes that the particular persistence of a toxin depends on its particular intracellular half-life, or stability, i.e., the longer the toxin is available in the cell, the longer the effect. Keller et al., FEBS Letters 456:137-42 (1999). Many factors contribute to the intracellular stability of a toxin, but primarily, the better it is able to resist the metabolic actions of intracellular proteases to break it down, the more stable it is. Erdal et al. Naunynschmiedeber's Arch. Pharmacol. 351:67-78 (1995).

In general, the ability of a molecule to resist metabolic actions of intracellular proteases may depend on its structures. For example, the primary structure of a molecule may include a unique primary sequence which may cause the molecule to be easily degraded by proteases or difficult to be degraded. For example, Varshavsky A. describes polypeptides terminating with certain amino acids are more susceptible to degrading proteases. Proc. Natl. Acad. Sci. USA 93:12142-12149 (1996).

Furthermore, intracellular enzymes are known to modify molecules, for example polypeptides through, for example, N-glycosylation, phosphorylation etc. this kind of modification will be referred to herein as "secondary modification". "Secondary modification" often refers to the modification of endogenous molecules, for example, polypeptides after they are translated from RNAs. However, as used herein, "secondary modification" may also refer to an enzyme's, for example an intracellular enzyme's, ability to modify exogenous molecules. For example, after a patient is administered with exogenous molecules, e.g. drugs, these molecules may undergo a secondary modification by the action of the patient's enzymes, for example intracellular enzymes.

WO 96/39166 describes analogues of Botulinum toxin and pharmaceutical compositions of botulinum toxin having longer half-life in neuromuscular tissue.

Kurazono et al. describe that there are minimal essential domains which are specific for toxicity of the light chains of tetanus toxin and botulinum neurotoxin type A.

WO 98/07864 describes recombinant botulinum toxin fragments having domains of the entire toxin.

Certain secondary modifications of molecules, for example polypeptides, may resist or facilitate the actions of degrading proteases. These secondary modifications may, among other things, (1) affect the ability of a degrading protease to act directly on the molecule and/or (2) affect the ability of the molecules to be sequestered into vesicles to be protected against these degrading proteases.

There is a need to have modified neurotoxins which have efficacies of the various botulinum toxin serotypes, but with altered biological persistence, and methods for preparing such toxins.

### Summary of the Invention

The present invention meets this need and provides for modified neurotoxins with altered biological persistence and explains methods for preparing such toxins.

Without wishing to be limited by any theory or mechanism of operation, it is believed that Botulinum toxins have secondary modification sites, which may determine their biological persistence. A "secondary modification site" as used herein means a location on a molecule, for example a particular fragment or a polypeptide, which may be targeted by an enzyme, for example an intra-cellular enzyme, to affect a modification to the site, for example phosphorylation, glycosylation, etc. The secondary modification, for example phosphorylation, may help resist or facilitate the actions of degrading proteases acting on the toxins, which in turn increase or decrease the persistence, or stability, of the toxins, respectively. Alternatively, it is believed that these secondary modification sites may prevent or facilitate the transporation of the toxin into vesicles to be protected from degrading proteases.

It is further believed that one of the roles of the secondary modification is to add to or take away the three dimensional and/or the chemical requirements necessary for protein interactions, for example between a molecule and a degrading protease, or a molecule and a vesicular transporter.

Therefore, a modified neurotoxin including a structural modification as described in claim 1 or 2 has an increased persistence as compared to an identical neurotoxin without the structural modification. The structural modification may include a partial or complete deletion or mutation of at least one modification site. The structural modification includes the addition of a certain modification site. The altered persistence is the enhancement of the biological persistence.

Preferably, the altered persistence is affected by the alteration in the stability of the modified neurotoxin.

For example, the light chain of BoNT/A has amino acid fragments for various secondary modification sites (hereinafter "modification sites") including, but not limited to, N-glycosylation, casein kinase II (CK-2) phosphorylation, N-terminal myristylation, protein kinase C (PKC) phosphorylation and tyrosine phosphorylation. BoNT/E also has these various secondary modification sites. The structural modification includes the addition of one or more of a modification site to a neurotoxin to form a modified neurotoxin, as defined in the claims.

This specification also provide for methods of producing modified neurotoxins. Additionally, this specification provide for methods of using the modified neurotoxins to treat biological disorders.

### Definitions

Before proceeding to describe the present invention, the following definitions are provided and apply herein.

"Heavy chain" means the heavy chain of a clostridial neurotoxin. It preferably has a molecular weight of about 100 kD and may be referred to herein as H chain or as H.

"H_{N}" means a fragment (preferably having a molecular weight of about 50 kD) derived from the H chain of a Clostridial neurotoxin which is approximately equivalent to the amino terminal segment of the H chain, or the portion corresponding to that fragment in the intact in the H chain. It is believed to contain the portion of the natural or wild type clostridial neurotoxin involved in the translocation of the L chain across an intracellular endosomal membrane.

"H_{C}" means a fragment (about 50 kD) derived from the H chain of a clostridial neurotoxin which is approximately equivalent to the carboxyl terminal segment of the H chain, or the portion corresponding to that fragment in the intact H chain. It is believed to be immunogenic and to contain the portion of the natural or wild type Clostridial neurotoxin involved in high affinity, presynaptic binding to motor neurons.

"Light chain" means the light chain of a clostridial neurotoxin. It preferably has a molecular weight of about 50 kD, and can be referred to as L chain, L or as the proteolytic domain (amino acid sequence) of a clostridial neurotoxin. The light chain is believed to be effective as an inhibitor of neurotransmitter release when it is released into a cytoplasm of a target cell.

"Neurotoxin" means a molecule that is capable of interfering with the functions of a neuron. The "neurotoxin" may be naturally occurring or man-made.

"Modified neurotoxin" means a neurotoxin which includes a structural modification. In other words, a "modified neurotoxin" is a neurotoxin which has been modified by a structural modification. The structural modification changes the biological persistence, preferably the biological half-life, of the modified neurotoxin relative to the neurotoxin from which the modified neurotoxin is made. The modified neurotoxin is structurally different from a naturally existing neurotoxin.

"Structural modification" means a physical change to the neurotoxin that may be affected by, for example, covalently fusing one or more amino acids to the neurotoxin. "Structural modification" also means the deletion of one or more amino acids from a neurotoxin. Furthermore, "structural modification" may also mean any changes to a neurotoxin that makes it physically or chemically different from an identical neurotoxin without the structural modification.

"Biological persistence" means the time duration in which a neurotoxin or a modified neurotoxin causes an interference with a neuronal function, for example the time duration in which a neurotoxin or a modified neurotoxin causes a substantial inhibition of the release of acetylcholine from a nerve terminal.

"Biological half-life" means the time that the concentration of a neurotoxin or a modified neurotoxin, preferably the active portion of the neurotoxin or modified neurotoxin, for example the light chain of botulinum toxins, is reduced to half of the original concentration in a mammal, preferably in the neurons of the mammal.

"Modification site" means a particular amino acid or a fragment of amino acids where upon secondary modification may takes place. "Modification site" may also mean a particular amino acid or a particular fragment of amino acids necessary for a certain secondary modification to occur.

### Detailed Description of the Invention

The present invention is, in part, based upon the discovery that the biological persistence of a neurotoxin may be altered by structurally modifying the neurotoxin. In other words, a modified neurotoxin with an altered biological persistence may be formed from a neurotoxin containing or including a structural modification. Preferably, the inclusion of the structural modification may alter the biological half-life of the modified neurotoxin. An altered biological persistence, preferably an altered biological half-life, means that the biological persistence (or biological half-life) of a modified neurotoxin is different from that of an identical neurotoxin without the structural modification. Additionally, the biological persistence, preferably the biological half-life, may be altered to be longer or shorter.

In a reference aspect, the structural modification includes a partial or complete deletion or mutation of the modification site of the neurotoxin to form a modified neurotoxin. The inclusion of the modification site enhances the biological persistence of the modified neurotoxin. Preferably, the partial or complete deletion, or mutation of the modification site enhances the biological half-life of the modified neurotoxin. More preferably, the biological half-life of the modified neurotoxin is enhanced by about 10%. Even more preferably, the biological half-life of the modified neurotoxin is enhanced by about 100%. Generally speaking, the modified neurotoxin has a biological persistence of about 20% to 300% more than an identical neurotoxin without the structural modification. That is, for example, the modified neurotoxin including the modified modification site is able to cause a substantial inhibition of acetylcholine release from a nerve terminal for about 20% to about 300% longer than a neurotoxin that is not modified.

In one reference aspect, the structural modification includes a partial or complete deletion or mutation of the modification site of the neurotoxin to form a modified neurotoxin. The inclusion of the modification site may reduce the biological persistence of the modified neurotoxin. Preferably, the partial or complete deletion, or mutation of the modification site reduces the biological half-life of the modified neurotoxin. More preferably, the biological half-life of the modified neurotoxin is reduced by about 10%. Even more preferably, the biological half-life of the modified neurotoxin is reduced by about 99%. Generally speaking, the modified neurotoxin has a biological persistence of about 20% to 300% less than an identical neurotoxin without the structural modification. That is, for example, the modified neurotoxin including the modified modification site is able to cause a substantial inhibition of acetylcholine release from a nerve terminal for about 20% to about 300% shorter in time than a neurotoxin that is not modified.

For example, BoNT/A and BoNT/E have the following potential secondary modification sites as shown on Tables 1 and 2, respectively.

**TABLE 1**

| N-Glycosylation (for reference) |
|---|
| 173-NLTR |
| 382-NYTI |
| 411-NFTK |
| 417-NFTG |
| |

| Casein kinase II (CR-2) phosphorylation sites (inventive) |
|---|
| 51-TNPE |
| 70-SYYD |
| 79-TDNE |
| 120-STID |
| 253-SGLE |
| 258-SFEE |
| 275-SLQE |
| 384-TIYD |
| |

| N-terminal myristylation sites (for reference): |
|---|
| 15-GVDIAY |
| 141-GSYRSE |
| 254-GLEVSF |
| |

| Protein kinase C (PKC) phosphorylation sites (inventive): |
|---|
| 142-SYR |
| 327-SGK |
| 435-TSK |
| |

| Tyrosine phosphorylation sites :(inventive): |
|---|
| 92-KLFERIY |
| 334-KLKFDKLY |

**TABLE 2**

| N-glycosylation (for reference): |
|---|
| 97-NLSG |
| 138-NGSG |
| 161-NSSN |
| 164-NISL |
| 365-NDSI |
| 370-NISE |
| |

| Casein kinase II (CK-2) phosphorylation sites (inventive): |
|---|
| 51-TPQD |
| 67-SYYD |
| 76-SDEE |
| 130-SAVE |
| 198-SMNE |
| 247-TNIE |
| 333-SFTE |
| 335-TEFD |
| |

| N-terminal myristylation sites (for reference): |
|---|
| 220-GLYGAK |
| 257-GTDLNI |
| 386-GQNANL |
| |

| Protein kinase C (PKC) phosphorylation sites :(inventive): |
|---|
| 60-SLK |
| 166-SLR |
| 191-SFR |
| 228-TTK |
| 234-TQK |
| 400-TGR |
| 417-SVK |
| |

| Tyrosine kinase phosphorylation sites (inventive): |
|---|
| 62-KNGDSSY |
| 300-KDVFEAKY |

In one preferred embodiment, one or more of the modification site of BoNT/A, is partially deleted, completely deleted or mutated, resulting in a modified neurotoxin with an altered biological persistence, preferably an altered biological half-life. In one embodiment, the modified neurotoxin is altered to have a longer biological persistence, preferably longer biological half-life. In another aspect, the modified neurotoxin is altered to have a shorter persistence, preferably a shorter biological half-life.

In one preferred embodiment, one or more of the modification site of BoNT/E, is partially deleted, completely deleted or mutated, resulting in a modified neurotoxin with an altered biological persistence, preferably an altered biological half-life. In one embodiment, the modified neurotoxin is altered to have a longer biological persistence, preferably longer biological half-life. In another aspect, the modified neurotoxin is altered to have a shorter persistence, preferably a shorter biological half-life as compared to an identical neurotoxin without the structural modification.

In one broad embodiment, the modified neurotoxin may include additional modification sites fused onto neurotoxins to form modified neurotoxins. The modification sites may be any modification sites known in the art, including the ones listed on Tables 1 and 2. Such inclusion of the modification site may enhance the biological persistence of the modified neurotoxin. Preferably, the modification site enhances the biological half-life of the modified neurotoxin. More preferably, the biological half-life of the modified neurotoxin is enhanced by about 10%. Even more preferably, the biological half-life of the modified neurotoxin is enhanced by about 100%. Generally speaking, the modified neurotoxin has a biological persistence of about 20% to 300% more than an identical neurotoxin without the structural modification. That is, for example, the modified neurotoxin including the modified site is able to cause a substantial inhibition of acetylcholine release from a nerve terminal for about 20% to about 300% longer than a neurotoxin that is not modified. A non-limiting example of a modified neurotoxin with an additional modification site is Bo/E with a casein kinase II phosphorylation site, preferably TDNE, fused to its primary structure. More preferably, the TDNE is fused to position 79 of BoNT/E or a position on BoNT/E which substantially corresponds to position 79 of BoNT/A.

In one broad embodiment, the modified neurotoxin may include additional modification sites fused onto neurotoxins to form modified neurotoxins. The modification sites may be any modification sites known in the art, including the ones listed on Tables 1 and 2. In one aspect, such inclusion of the modification site may reduce the biological persistence of the modified neurotoxin. Preferably, the modification site reduces the biological half-life of the modified neurotoxin. More preferably, the biological half-life of the modified neurotoxin is reduced by about 10%. Even more preferably, the biological half-life of the modified neurotoxin is reduced by about 99%. Generally speaking, the modified neurotoxin has a biological persistence of about 20% to 300% less than an identical neurotoxin without the structural modification. That is, for example, the modified neurotoxin including the modified site is able to cause a substantial inhibition of acetylcholine release from a nerve terminal for about 20% to about 300% shorter in time than a neurotoxin that is not modified. A non-limiting example of a modified neurotoxin with an additional modification site is Bo/A with a casein kinase II phosphorylation site, preferably SDEE, fused to its primary structure. More preferably, the SDEE is fused to position 76 of BoNT/A or a position on BoNT/A which substantially corresponds to position 76 of BoNT/E.

In one embodiment, the structural modification may include the addition and the partial or complete deletion or mutation of modification sites. For example, a modified neurotoxin may be BoNT/A with GVDIAY at position 15 deleted and includes a SLK fragment for protein kinase C phosphorylation. The SLK fragment is preferably fused to position 60 of BoNT/A or a position on BoNT/A which substantially corresponds to position 60 of BoNT/E. The modified neurotoxin according to this embodiment may have altered biological persistence. In one embodiment, the biological persistence is increased. Preferably, the modified neurotoxin according to this embodiment may have altered biological half-life. In one embodiment, the biological half-life is increased. In another aspect, the biological half-life is decreased.

In one broad aspect of the present specification, a method is provided for treating a biological disorder using a modified neurotoxin. The treatments may include treating neuromuscular disorders, autonomic nervous system disorders and pain.

The neuromuscular disorders and conditions that may be treated with a modified neurotoxin include: for example, strabismus, blepharospasm, spasmodic torticollis (cervical dystonia), oromandibular dystonia and spasmodic dysphonia (laryngeal dystonia).

For example, Borodic U.S. Patent No. 5,053,005 discloses methods for treating juvenile spinal curvature, i.e. scoliosis, using BoNT/A. Using substantially similar methods as disclosed by Borodic, a modified neurotoxin can be administered to a mammal, preferably a human, to treat spinal curvature. In a preferred embodiment, a modified neurotoxin comprising BoNT/E fused with an N-terminal myristylation site is used. Even more preferably, a modified neurotoxin comprising BoNT/E with an N-terminal myristylation site fused to position 15 of its light chain, or a position substantially corresponding to position 15 of the BoNT/A light chain, is used to prepare a pharmaceutical for the treatment of a mammal, preferably a human, to treat spinal curvature. The modified neurotoxin used to prepare a pharmaceutical may be administered to treat other neuromuscular disorders using well known techniques that are commonly performed with BoNT/A.

Autonomic nervous system disorders may also be treated with a modified neurotoxin. For example, glandular malfunctioning is an autonomic nervous system disorder. Glandular malfunctioning includes excessive sweating and excessive salivation. Respiratory malfunctioning is another example of an autonomic nervous system disorder. Respiratory malfunctioning includes chronic obstructive pulmonary disease and asthma. Sanders et al. discloses methods for treating the autonomic nervous system, such as excessive sweating, excessive salivation, asthma, etc., using naturally existing botulinum toxins.

In one embodiment, substantially similar methods to that of Sanders et al. may be employed, but using a codified neurotoxin, to treat autonomic nervous system disorders such as the ones discussed above. For example, a modified neurotoxin may be locally applied to the nasal cavity of the mammal in an amount sufficient to degenerate cholinergic neurons of the autonomic nervous system that control the mucous secretion in the nasal cavity.

Pain that may be treated by a modified neurotoxin includes pain caused by muscle tension, or spasm, or pain that is not associated with muscle spasm. For example, Binder in U.S. Patent No. 5,714,468 discloses that headache caused by vascular disturbances, muscular tension, neuralgia and neuropathy may be treated with a naturally occurring botulinum toxin, for example BoNT/A. Substantially similar methods to that of Binder may be employed, but using a modified neurotoxin, to treat headache, especially the ones caused by vascular disturbances, muscular tension, neuralgia and neuropathy. Pain caused by muscle spasm may also be treated by an administration of a modified neurotoxin. For example, a modified neurotoxin comprising BoNT/E with an N-terminal myristylation site fused to position 15 of its light chain, or a position substantially corresponding to position 15 of the BoNT/A light chain, may be administered intramuscularly at the pain/spasm location to alleviate pain.

Furthermore, a modified neurotoxin may be administered to a mammal to treat pain that is not associated with a muscular disorder, such as spasm. In one broad embodiment, methods of the present specification to treat non-spasm related pain include central administration or peripheral administration of the modified neurotoxin.

For example, Foster et al. in U.S. Patent No. 5,989,545 discloses that a botulinum toxin conjugated with a targeting moiety may be administered centrally (intrathecally) to alleviate pain. Substantially similar methods to.that of Foster et al. may be employed, but using the modified neurotoxin according to this invention, to treat pain. The pain to be treated may be an acute pain, or preferably, chronic pain.

An acute or chronic pain that is not associated with a muscle spasm may also be alleviated with a local, peripheral administration of the modified neurotoxin to an actual or a perceived pain location on the mammal. The modified neurotoxin may be administered subcutaneously at or near the location of pain, for example at or near a cut. Alternatively, the modified neurotoxin can be administered intramuscularly at or near the location of pain, for example at or near a bruise location on the mammal. Further, the modified neurotoxin may be injected directly into a joint of a mammal, for treating or alleviating pain cause arthritis conditions. Also, frequent repeated injections or infusion of the modified neurotoxin to a peripheral pain location is possible. However, given the long lasting therapeutic effects of the present invention, frequent injections or infusion of the neurotoxin may not be necessary. For example, practice of the present invention can provide an analgesic effect, per injection, for 2 months or longer, for example 27 months, in humans.

Without wishing to limit the invention to any mechanism or theory of operation, it is believed that when the modified neurotoxin is administered locally to a peripheral location, it inhibits the release of neuro-substances, for example substance P, from the peripheral primary sensory terminal. Since the release of substance P by the peripheral primary sensory terminal may cause or at least amplify pain transmission process, inhibition of its release at the peripheral primary sensory terminal will dampen the transmission of pain signals from reaching the brain.

In addition to having pharmacologic actions at the peripheral location, the modified neurotoxin of the present invention may also have inhibitory effects in the central nervous system. Presumably the retrograde transport is via the primary afferent. This hypothesis is supported by our experimental data which shows that BoNT/A is retrograde transported to the dorsal horn when the Neurotoxin is injected peripherally. Moreover, work by Weigand et al, Nauny-Schmiedeberg's Arch. Pharmacol. 1976; 292, 161-165, and Habermann, Nauny-Schmiedeberg's Arch. Pharmacol. 1974; 281, 47-56, showed that botulinum toxin is able to ascend to the spinal area by retrograde transport. As such, a modified neurotoxin, for example BoNT/A with one or more amino acids deleted from the leucine-based motif, injected at a peripheral location, for example intramuscularly, may be retrograde transported from the peripheral primary sensory terminal to the central primary sensory terminal.

The amount of the modified neurotoxin administered can vary widely according to the particular disorder being treated, its severity and other various patient variables including size, weight, age, and responsiveness to therapy. Generally, the dose of modified neurotoxin to be administered will vary with the age, presenting condition and weight of the mammal, preferably a human, to be treated. The potency of the modified neurotoxin will also be considered.

Assuming a potency which is substantially equivalent to LD₅₀ = 2,730 U in a human patient and an average person is 75kg, a lethal dose would be about 36 U/kg of a modified neurotoxin. Therefore, when a modified neurotoxin with such an LD₅₀ is administered, it would be appropriate to administer less than 36 U/kg of the modified neurotoxin into human subjects. Preferably, about 0.01 U/kg to 30 U/kg of the modified neurotoxin is administered. More preferably, about 1 U/kg to about 15 U/kg of the modified neurotoxin is administered. Even more preferably, about 5 U/kg to about 10 U/kg modified neurotoxin is administered. Generally, the modified neurotoxin will be administered as a composition at a dosage that is proportionally equivalent to about 2.5 cc/100 U. Those of ordinary skill in the art will know, or can readily ascertain, how to adjust these dosages for neurotoxin of greater or lesser potency.

Although examples of routes of administration and dosages are provided, the appropriate route of administration and dosage are generally determined on a case by case basis by the attending physician. Such determinations are routine to one of ordinary skill in the art (see for example, Harrison's Principles of Internal Medicine (1998), edited by Anthony Fauci et al., 14th edition, published by McGraw Hill). For example, the route and dosage for administration of a modified neurotoxin according to the present disclosed invention can be selected based upon criteria such as the solubility characteristics of the modified neurotoxin chosen as well as the types of disorder being treated.

The modified neurotoxin may be produced by chemically linking the modification sites to a neurotoxin using conventional chemical methods well known in the art. The neurotoxin may be obtained from harvesting neurotoxins. For example, BoNT/E can be obtained by establishing and growing cultures of Clostridium botulinum in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the BoNT/B serotype only cleave a portion of the toxin produced. The exact proportion of nicked to unnicked molecules depends on the length of incubation and the temperature of the culture. Therefore, a certain percentage of any preparation of, for example, the BoNT/B toxin is likely to be inactive, possibly accounting for the known significantly lower potency of BoNT/B as compared to BoNT/A. The presence of inactive botulinum toxin molecules in a clinical preparation will contribute to the overall protein load of the preparation, which has been linked to increased antigenicity, without contributing to its clinical efficacy. Additionally, it is known that BoNT/B has, upon intramuscular injection, a shorter duration of activity and is also less potent than BoNT/A at the same dose level.

The modified neurotoxin may also be produced by recombinant techniques. Recombinant techniques are preferable for producing a neurotoxin having amino acid sequence regions from different Clostridial species or having modified amino acid sequence regions. Also, the recombinant technique is preferable in producing BoNT/A with the modified (deleted or mutated) or added modification sites. The technique includes steps of obtaining genetic materials from natural sources, or synthetic sources, which have codes for a neuronal binding moiety, an amino acid sequence effective to translocate the neurotoxin or a part thereof, and an amino acid sequence having therapeutic activity when released into a cytoplasm of a target cell, preferably a neuron. In a preferred embodiment, the genetic materials have codes for the biological persistence enhancing component, preferably the leucine-based motif, the H_{c}, the H_{N} and the L chain of the Clostridial neurotoxins and fragments thereof. The genetic constructs are incorporated into host cells for amplification by first fusing the genetic constructs with a cloning vectors, such as phages or plasmids. Then the cloning vectors are inserted into hosts, preferably E. coli's. Following the expressions of the recombinant genes in host cells, the resultant proteins can be isolated using conventional techniques.

There are many advantages to producing these modified neurotoxins recombinantly. For example, to form a modified neurotoxin, a modifying fragment must be attached or inserted into a neurotoxin. The production of neurotoxin from anaerobic Clostridium cultures is a cumbersome and time-consuming process including a multistep purification protocol involving several protein precipitation steps and either prolonged and repeated crystallization of the toxin or several stages of column chromatography. Significantly, the high toxicity of the product dictates that the procedure must be performed under strict containment (BL-3). During the fermentation process, the folded single-chain neurotoxins are activated by endogenous clostridial proteases through a process termed nicking to create a dichain. Sometimes, the process of nicking involves the removal of approximately 10 amino acid residues from the single-chain to create the dichain form in which the two chains remain covalently linked through the intrachain disulfide bond.

The nicked neurotoxin is much more active than the unnicked form. The amount and precise location of nicking varies with the serotypes of the bacteria producing the toxin. The differences in single-chain neurotoxin activation and, hence, the yield of nicked toxin, are due to variations in the serotype and amounts of proteolytic activity produced by a given strain. For example, greater than 99% of *Clostridial botulinum* serotype A single-chain neurotoxin is activated by the Hall A *Clostridial botulinum* strain, whereas serotype B and E strains produce toxins with lower amounts of activation (0 to 75% depending upon the fermentation time). Thus, the high toxicity of the mature neurotoxin plays a major part in the commercial manufacture of neurotoxins as therapeutic agents.

The degree of activation of engineered clostridial toxins is, therefore, an important consideration for manufacture of these materials. It would be a major advantage if neurotoxins such as botulinum toxin and tetanus toxin could be expressed, recombinantly, in high yield in rapidly-growing bacteria (such as heterologous *E. coli* cells) as relatively non-toxic single-chains (or single chains having reduced toxic activity) which are safe, easy to isolate and simple, to convert to the fully-active form.

With safety being a prime concern, previous work has concentrated on the expression in *E.coli* and purification of individual H and L chains of tetanus and botulinum toxins; these isolated chains are, by themselves, non-toxic; see Li et al., Biochemistry 33:7014-7020 (1994); Zhou et al., Biochemistry 34:15175-15181 (1995). Following the separate production of these peptide chains and under strictly controlled conditions the H and L chains can be combined by oxidative disulphide linkage to form the neuroparalytic di-chains(di-polypeptide), linked together by a disulfide bond. Preferably one of the polypeptides is a Clostridial neurotoxin heavy chain and the other is a Clostridial neurotoxin light chain. The neuronal binding moiety is preferably part of the heavy chain.

### EXAMPLES

The following non-limiting examples provide those of ordinary skill in the art with specific preferred methods to treat non-spasm related pain within the scope of the present invention and are not intended to limit the scope of the invention.

### Example 1

### Treatment of Pain Associated with Muscle Disorder

An unfortunate 36 year old woman has a 15 year history of temporomandibular joint disease and chronic pain along the masseter and temporalis muscles. Fifteen years prior to evaluation she noted increased immobility of the jaw associated with pain and jaw opening and closing and tenderness along each side of her face. The left side is originally thought to be worse than the right. She is diagnosed as having temporomandibular joint (TMJ) dysfunction with subluxation of the joint and is treated with surgical orthoplasty meniscusectomy and condyle resection.

She continues to have difficulty with opening and closing her jaw after the surgical procedures and for this reason, several years later, a surgical procedure to replace prosthetic joints on both sides is performed. After the surgical procedure progressive spasms and deviation of the jaw ensues. Further surgical revision is performed subsequent to the original operation to correct prosthetic joint loosening. The jaw continues to exhibit considerable pain and immobility after these surgical procedures. The TMJ remained tender as well as the muscle itself. There are tender points over the temporomandibular joint as well as increased tone in the entire muscle. She is diagnosed as having post-surgical myofascial pain syndrome and is injected with about 8 U/kg to about 15 U/kg of the modified neurotoxin into the masseter and temporalis muscles, preferably the modified neurotoxin comprises BoNT/E with an N-terminal myristylation site, for example GVDIAY, fused to position 15 of its light chain, or a position substantially corresponding to position 15 of the BoNT/A light chain.

Several days after the injections she noted substantial improvement in her pain and reports that her jaw feels looser. This gradually improves over a 2 to 3 week period in which she notes increased ability to open the jaw and diminishing pain. The patient states that the pain is better than at any time in the last 4 years. The improved condition persists for up to 27 months after the original injection of the modified neurotoxin.

### Example 2

### Treatment of Pain Subsequent to Spinal Cord Injury

A patient, age 39, experiencing pain subsequent to spinal cord injury is treated by intrathecal administration, for example by spinal tap or by catherization (for infusion), to the spinal cord, with about 0.1 U/kg to about 10 U/kg of the modified neurotoxin, preferably the modified neurotoxin comprises BoNT/E with an N-terminal myristylation site, for example GVDIAY, fused to position 15 of its light chain, or a position substantially corresponding to position 15 of the BoNT/A light chain. The particular toxin dose and site of injection, as well as the frequency of toxin administrations depend upon a variety of factors within the skill of the treating physician, as previously set forth. Within about 1 to about 7 days after the modified neurotoxin administration, the patient's pain is substantially reduced. The pain alleviation persists for up to 27 months.

### Example 3

### Peripheral Administration of a Modified Neurotoxin to Treat "Shoulder-Hand Syndrome"

Pain in the shoulder, arm, and hand can develop, with muscular dystrophy, osteoporosis, and fixation of joints. While most common after coronary insufficiency, this syndrome may occur with cervical osteoarthritis or localized shoulder disease, or after any prolonged illness that requires the patient to remain in bed.

A 46 year old woman presents a shoulder-hand syndrome type pain. The pain is particularly localized at the deltoid region. The patient is treated by a bolus injection of about 0.05 U/kg to about 2 U/kg of a modified neurotoxin subcutaneously to the shoulder, preferably the modified neurotoxin comprises BoNT/E with an N-terminal myristylation site, for example GVDIAY, fused to position 15 of its light chain, or a position substantially corresponding to position 15 of the BoNT/A light chain. The particular dose as well as the frequency of administrations depends upon a variety of factors within the skill of the treating physician, as previously set forth. Within 1-7 days after modified neurotoxin administration the patient's pain is substantially alleviated. The duration of the pain alleviation is from about 7 to about 27 months.

### Example 4

### Peripheral Administration of a Modified Neurotoxin to Treat Postherpetic Neuralgia

Postherpetic neuralgia is one of the most intractable of chronic pain problems. Patients suffering this excruciatingly painful process often are elderly, have debilitating disease, and are not suitable for major interventional procedures. The diagnosis is readily made by the appearance of the healed lesions of herpes and by the patient's history. The pain is intense and emotionally distressing. Postherpetic neuralgia may occur anywhere, but is most often in the thorax.

A 76 year old man presents a postherpetic type pain. The pain is localized to the abdomen region. The patient is treated by a bolus injection of between about 0.05 U/kg to about 2 U/kg of a modified neurotoxin intradermally to the abdomen, preferably the modified neurotoxin comprises BoNT/E with an N-terminal myristylation site, for example GVDIAY, fused to position 15 of its light chain, or a position substantially corresponding to position 15 of the BoNT/A light chain. The particular dose as well as the frequency of administrations depends upon a variety of factors within the skill of the treating physician, as previously set forth. Within 1-7 days after modified neurotoxin administration the patient's pain is substantially alleviated. The duration of the pain alleviation is from about 7 to about 27 months.

### Example 5

### Peripheral Administration of a Modified Neurotoxin to Treat Nasopharyngeal Tumor Pain

These tumors, most often squamous cell carcinomas, are usually in the fossa of Rosenmuller and may invade the base of the skull. Pain in the face is common. It is constant, dull-aching in nature.

A 35 year old man presents a nasopharyngeal tumor type pain. Pain is found at the lower left cheek. The patient is treated by a bolus injection of between about 0.05 U/kg to about 2 U/kg of a modified neurotoxin intramuscularly to the cheek, preferably the modified neurotoxin comprises BoNT/E with an N-terminal myristylation site, for example GVDIAY, fused to position 15 of its light chain, or a position substantially corresponding to position 15 of the BoNT/A light chain. The particular dose as well as the frequency of administrations depends upon a variety of factors within the skill of the treating physician, as previously set forth. Within 1-7 days after modified neurotoxin administration the patient's pain is substantially alleviated. The duration of the pain alleviation is from about 7 to about 27 months.

### Example 6

### Peripheral Administration of a Modified Neurotoxin to Treat Inflammatory Pain

A patient, age 45, presents an inflammatory pain in the chest region. The patient is treated by a bolus injection of between about 0.05 U/kg to about 2 U/kg of a modified neurotoxin intramuscularly to the chest, preferably the modified neurotoxin comprises BoNT/E with an N-terminal myristylation site, for example GVDIAY, fused to position 15 of its light chain, or a position substantially corresponding to position 15 of the BoNT/A light chain. The particular dose as well as the frequency of administrations depends upon a variety of factors within the skill of the treating physician, as previously set forth. Within 1-7 days after modified neurotoxin administration the patient's pain is substantially alleviated. The duration of the pain alleviation is from about 7 to about 27 months.

### Example 7

### Treatment of Excessive Sweating

A male, age 65, with excessive unilateral sweating is treated by administering 0.05 U/kg to about 2 U/kg of a modified neurotoxin, depending upon degree of desired effect. Preferably the modified neurotoxin comprises BoNT/E with an N-terminal myristylation site, for example GVDIAY, fused to position 15 of its light chain, or a position substantially corresponding to position 15 of the BoNT/A light chain. The administration is to the gland nerve plexus, ganglion, spinal cord or central nervous system. The specific site of administration is to be determined by the physician's knowledge of the anatomy and physiology of the target glands and secretary cells. In addition, the appropriate spinal cord level or brain area can be injected with the toxin. The cessation of excessive sweating after the modified neurotoxin treatment is up to 27 months.

### Example 8

### Post Surgical Treatments

A female, age 22, presents a torn shoulder tendon and undergoes orthopedic surgery to repair the tendon. After the surgery, the patient is administered intramuscularly with about 0.05 U/kg to about 2 U/kg of a modified neurotoxin to the shoulder. Preferably, the modified neurotoxin comprises BoNT/A with an N-terminal myristylation site, for example GLEVSF at position 254, deleted. The specific site of administration is to be determined by the physician's knowledge of the anatomy and physiology of the muscles. The administered modified neurotoxin reduces movement of the arm to facilitate the recovery from the surgery. The effect of the modified neurotoxin is for about five weeks.

### Example 9

### Treatment of Spasmodic Dysphonia

A male, age 45, unable to speak clearly, due to spasm of the vocal chords, is treated by injection of the vocal chords with a bout 0.1 U/kg to about 2 U/kg of modified neurotoxins according to the present invention. After 3-7 days, the patient is able to speak clearly. The patient's condition is alleviated for about 7 months to about 27 months.

### Example 10

### Treatment of Spasmodic Torticollis

A male, age 45, suffering from spasmodic torticollis, as manifested by spasmodic or tonic contractions of the neck musculature, producing stereotyped abnormal deviations of the head, the chin being rotated to the side, and the shoulder being elevated toward the side at which the head is rotated, is treated by injection with about 8 U/kg to about 15 U/kg of neurotoxins according to the present invention. After 3-7 days, the symptoms are substantially alleviated; i.e., the patient is able to hold his head and shoulder in a normal position. The alleviation persists for about 7 months to about 27 months.

### Example 11

### Treatment of Essential Tremor

A male, age 45, suffering from essential tremor, which is manifested as a rhythmical oscillation of head or hand muscles and is provoked by maintenance of posture or movement, is treated by injection with about 8 U/kg to about 15 U/kg of modified neurotoxin of the present invention. After two to eight weeks, the symptoms are substantially alleviated; i.e., the patient's head or hand ceases to oscillate. The symptoms are alleviated for about 5 months to about 27 months.

### Example 12

### Production of a Modified Neurotoxin with an Altered Biological Persistence

A modified neurotoxin according to the present invention may be produced with recombinant techniques. An example of a recombinant technique is one which includes the step of obtaining genetic materials from oligonucleotide sequences having codes for a modified neurotoxin according to the present invention. The genetic constructs are incorporated into host cells for amplification by first fusing the genetic constructs with a cloning vectors, such as phages or plasmids. Then the cloning vectors are inserted into' hosts, preferably E. coli's. Following the expressions of the recombinant genes in host cells, the resultant proteins can be isolated using conventional techniques. See also International Patent Application WO95/32738, the disclosure of which is incorporated in its entirety by reference herein.

The modified neurotoxin produced according to this example has an altered biological persistence. Preferably, the biological persistence is enhanced, more preferably enhanced by about 20% to about 300% relative to an identical neurotoxin without a leucine-based motif.

Although the present invention has been described in detail with regard to certain preferred methods, other embodiments, versions, and modifications within the scope of the present invention are possible. For example, a wide variety of modified neurotoxins can be effectively used in the methods of the present invention in place of clostridial neurotoxins. Additionally, the present invention enables peripheral administration methods wherein two or more modified neurotoxins, for example BoNT/E fused with a modification site and BoNT/B fused with a modification site, are administered concurrently or consecutively. Furthermore, a "targeting component" may be added to or substituted onto a modified neurotoxin of this invention. The "targeting component" may be a small molecule or a polypeptide having selective binding to a particular receptor. As such, a modified neurotoxin of the present invention comprising a targeting component may be specifically directed to a specific target receptor. See Foster et al in U.S. Patent No. 5,989,545 and Donovan in U.S. Patent Application Ser. No. 09/489,667.

## Claims

1. A modified BoNT/A neurotoxin comprising a BoNT/A neurotoxin including a structural modification, wherein the structural modification is effective to increase the biological persistence of the modified neurotoxin relative to an identical neurotoxin without the structural modification, and wherein the modified neurotoxin is structurally different from a naturally occurring neurotoxin
wherein the structural modification includes the presence of one or more secondary modification sites in addition to the ones that are already naturally present and
wherein the secondary modification site is a member selected from the group consisting of casein kinase II (CK-2) phosphorylation, protein kinase C (PKC) phosphorylation and tyrosine phosphorylation sites,
wherein the additional casein kinase II phosphorylation site is SEQ ID NO:5, SEQ ID NO: 6, SEQ ID NO: 7, SEQ ID NO: 8, SEQ ID NO: 9, SEQ ID NO: 10, SEQ ID NO: 11 or SEQ ID NO: 12, and
wherein the additional protein kinase C (PKC) phosphorylation site is SEQ ID NO: 16, SEQ ID NO: 17 or SEQ ID NO: 18 and
wherein the additional tyrosine phosphorylation site is SEQ ID NO: 19 or SEQ ID NO: 20.

2. A modified BoNT/E neurotoxin comprising a BoNT/E neurotoxin including a structural modification, wherein the structural modification is effective to increase the biological persistence of the modified neurotoxin relative to an identical neurotoxin without the structural modification, and wherein the modified neurotoxin is structurally different from a naturally occurring neurotoxin
wherein the structural modification includes the presence of one or more secondary modification sites in addition to the ones that are already naturally present and
wherein the secondary modification site is a member selected from the group consisting of casein kinase II (CK-2) phosphorylation, protein kinase C (PKC) phosphorylation and tyrosine phosphorylation sites,
wherein the additional casein kinase II phosphorylation site is SEQ ID NO: 27, SEQ ID NO: 28, SEQ ID NO: 29, SEQ ID NO: 30, SEQ ID NO: 31, SEQ ID NO: 32, SEQ ID NO: 33 or SEQ ID NO: 34, and
wherein the additional protein C kinase C (PKC) phosphorylation site is SEQ ID NO: 38, SEQ ID NO: 39, SEQ ID NO: 40, SEQ ID NO: 41, SEQ ID NO: 42, SEQ ID NO: 43 or SEQ ID NO: 44, and
wherein the additional tyrosine phosphorylation site is SEQ ID NO: 45 or SEQ ID NO: 46.

## Patentansprüche

1. Modifiziertes BoNT/A-Neurotoxin, umfassend ein BoNT/A-Neurotoxin, das eine strukturelle Modifikation aufweist, wobei die strukturelle Modifikation wirksam ist, die biologische Ausdauer des modifizierten Neurotoxins gegenüber einem identischen Neurotoxin ohne die strukturelle Modifikation zu erhöhen, und wobei das modifizierte Neurotoxin sich strukturell von einem natürlich auftretenden Neurotoxin unterscheidet,
wobei die strukturelle Modifikation die Gegenwart einer oder mehrerer sekundärer Modifikationsstellen zusätzlich zu denjenigen, die bereits natürlich vorhanden sind, aufweist, und
wobei die sekundäre Modifikationsstelle ein Mitglied ausgewählt aus der Gruppe bestehend aus Caseinkinase II (CK-2)-Phosphorylierungs-, Proteinkinase C (PKC)-Phosphorylierungs- und Tyrosinphosphorylierungsstelle ist,
wobei die zusätzliche Caseinkinase II-Phosphorylierungsstelle SEQ ID NO: 5, SEQ ID NO: 6, SEQ ID NO: 7, SEQ ID NO: 8, SEQ ID NO: 9, SEQ ID NO: 10, SEQ ID NO: 11 oder SEQ ID NO: 12 ist, und
wobei die zusätzliche Proteinkinase C (PKC)-Phosphorylierungsstelle SEQ ID NO: 16, SEQ ID NO: 17 oder SEQ ID NO: 18 ist, und
wobei die zusätzliche Tyrosinphosphorylierungsstelle SEQ ID NO: 19 oder SEQ ID NO: 20 ist.

2. Modifiziertes BoNT/A-Neurotoxin, umfassend ein BoNT/A-Neurotoxin, das eine strukturelle Modifikation aufweist, wobei die strukturelle Modifikation wirksam ist, die biologische Ausdauer des modifizierten Neurotoxins gegenüber einem identischen Neurotoxin ohne die strukturelle Modifikation zu erhöhen, und wobei das modifizierte Neurotoxin sich strukturell von einem natürlich auftretenden Neurotoxin unterscheidet,
wobei die strukturelle Modifikation die Gegenwart einer oder mehrerer sekundärer Modifikationsstellen zusätzlich zu denjenigen, die bereits natürlich vorhanden sind, aufweist, und
wobei die sekundäre Modifikationsstelle ein Mitglied ausgewählt aus der Gruppe bestehend aus Caseinkinase II (CK-2)-Phosphorylierungs-, Proteinkinase C (PKC)-Phosphorylierungs- und Tyrosinphosphorylierungsstelle ist,
wobei die zusätzliche Caseinkinase II-Phosphorylierungsstelle SEQ ID NO: 27, SEQ ID NO: 28, SEQ ID NO: 29, SEQ ID NO: 30, SEQ ID NO: 31, SEQ ID NO: 32, SEQ ID NO: 33 oder SEQ ID NO: 34 ist, und
wobei die zusätzliche Proteinkinase C (PKC)-Phosphorylierungsstelle SEQ ID NO: 38, SEQ ID NO: 39, SEQ ID NO: 40, SEQ ID NO: 41, SEQ ID NO: 42, SEQ ID NO: 43 oder SEQ ID NO: 44 ist, und
wobei die zusätzliche Tyrosinphosphorylierungsstelle SEQ ID NO: 45 oder SEQ ID NO: 46 ist.

## Revendications

1. Neurotoxine BoNT/A modifiée comprenant une neurotoxine BoNT/A incluant une modification structurale, dans laquelle la modification structurale est efficace pour faire augmenter la persistance biologique de la neurotoxine modifiée par rapport à une neurotoxine identique sans la modification structurale, et dans laquelle la neurotoxine modifiée est structuralement différente d'une neurotoxine naturelle dans laquelle la modification structurale comprend la présence d'un ou de plusieurs sites de modification secondaires outre les sites qui sont déjà naturellement présents et
dans laquelle le site de modification secondaire est un élément choisi dans le groupe constitué par les sites de phosphorylation de la caséine kinase II (CK-2), de phosphorylation de la protéine kinase C (PKC) et de phosphorylation de la tyrosine, dans laquelle le site de phosphorylation de la caséine kinase II supplémentaire est SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, et
dans laquelle le site de phosphorylation de la protéine kinase C (PKC) supplémentaire est SEQ ID NO : 16, SEQ ID NO : 17 ou SEQ ID NO : 18 et
dans laquelle le site de phosphorylation de la tyrosine supplémentaire est SEQ ID NO : 19 ou SEQ ID NO : 20.

2. Neurotoxine BoNT/E modifiée comprenant une neurotoxine BoNT/E incluant une modification structurale, dans laquelle la modification structurale est efficace pour faire augmenter la persistance biologique de la neurotoxine modifiée par rapport à une neurotoxine identique sans la modification structurale, et dans laquelle la neurotoxine modifiée est structuralement différente d'une neurotoxine naturelle, dans laquelle la modification structurale comprend la présence d'un ou de plusieurs sites de modification secondaires outre les sites qui sont déjà naturellement présents et
dans laquelle le site de modification secondaire est un élément choisi dans le groupe constitué par les sites de phosphorylation de la caséine kinase II (CK-2), de phosphorylation de la protéine kinase C (PKC) et de phosphorylation de la tyrosine, dans laquelle le site de phosphorylation de la caséine kinase II supplémentaire est SEQ ID NO : 27, SEQ ID NO : 28, SEQ ID NO : 29, SEQ ID NO : 30, SEQ ID NO : 31, SEQ ID NO : 32, SEQ ID NO : 33 ou SEQ ID NO : 34, et
dans laquelle le site de phosphorylation de la protéine kinase C (PKC) supplémentaire est SEQ ID NO : 38, SEQ ID NO : 39, SEQ ID NO : 40, SEQ ID NO : 41, SEQ ID NO : 42, SEQ ID NO : 43 ou SEQ ID NO : 44, et
dans laquelle le site de phosphorylation de la tyrosine supplémentaire est SEQ ID NO : 45 ou SEQ ID NO : 46.


### SEQUENCE LISTING

<110> Steward, Lance Spanoyannis, Athena Lin, Wei-Lin Aoki, Kei R
<120> Modified Clostrial Neurotoxins with Altered Biological Persistence
<130> 2916p
<140> not yet assigned
   <141> 2000-11-16
<160> 46
<170> PatentIn Ver. 2.1
<210> 1
   <211> 4
   <212> PRT
   <213> botulinum toxin
<400> 1
   Asn Leu Thr Arg
   1
<210> 2
   <211> 4
   <212> PRT
   <213> botulinum toxin
<400> 2
   Asn Tyr Thr Ile
   1
<210> 3
   <211> 4
   <212> PRT
   <213> botulinum toxin
<400> 3
   Asn Phe Thr Lys
   1
<210> 4
   <211> 4
   <212> PRT
   <213> botulinum toxin
<400> 4
   Asn Phe Thr Gly
   1
<210> 5
   <211> 4
   <212> PRT
   <213> botulinum toxin
<400> 5
   Thr Asn Pro Glu
   1
<210> 6
   <211> 4
   <212> PRT
   <213> botulinum toxin
<400> 6
   Ser Tyr Tyr Asp
   1
<210> 7
   <211> 4
   <212> PRT
   <213> botulinum toxin
<400> 7
   Thr Asp Asn Glu
   1
<210> 8
   <211> 4
   <212> PRT
   <213> botulinum toxin
<400> 8
   Ser Thr Ile Asp
   1
<210> 9
   <211> 4
   <212> PRT
   <213> botulinum toxin
<400> 9
   Ser Gly Leu Glu
   1
<210> 10
   <211> 4
   <212> PRT
   <213> botulinum toxin
<400> 10
   Ser Phe Glu Glu
   1
<210> 11
   <211> 4
   <212> PRT
   <213> botulinum toxin
<400> 11
   Ser Leu Gln Glu
   1
<210> 12
   <211> 4
   <212> PRT
   <213> botulinum toxin
<400> 12
   Thr Ile Tyr Asp
   1
<210> 13
   <211> 6
   <212> PRT
   <213> botulinum toxin
<400> 13
   Gly Val Asp Ile Ala Tyr
   1 5
<210> 14
   <211> 6
   <212> PRT
   <213> botulinum toxin
<400> 14
   Gly Ser Tyr Arg Ser Glu
   1 5
<210> 15
   <211> 6
   <212> PRT
   <213> botulinum toxin
<400> 15
   Gly Leu Glu Val Ser Phe
   1 5
<210> 16
   <211> 3
   <212> PRT
   <213> botulinum toxin
<400> 16
   Ser Tyr Arg
   1
<210> 17
   <211> 3
   <212> PRT
   <213> botulinum toxin
<400> 17
   Ser Gly Lys
   1
<210> 18
   <211> 3
   <212> PRT
   <213> botulinum toxin
<400> 18
   Thr Ser Lys
   1
<210> 19
   <211> 7
   <212> PRT
   <213> botulinum toxin
<400> 19
   Lys Leu Phe Glu Arg Ile Tyr
   1 5
<210> 20
   <211> 8
   <212> PRT
   <213> botulinum toxin
<400> 20
   Lys Leu Lys Phe Asp Lys Leu Tyr
   1 5
<210> 21
   <211> 4
   <212> PRT
   <213> botulinum toxin
<400> 21
   Asn Leu Ser Gly
   1
<210> 22
   <211> 4
   <212> PRT
   <213> botulinum toxin
<400> 22
   Asn Gly Ser Gly
   1
<210> 23
   <211> 4
   <212> PRT
   <213> botulinum toxin
<400> 23
   Asn Ser Ser Asn
   1
<210> 24
   <211> 4
   <212> PRT
   <213> botulinum toxin
<400> 24
   Asn Ile Ser Leu
   1
<210> 25
   <211> 4
   <212> PRT
   <213> botulinum toxin
<400> 25
   Asn Asp Ser Ile
   1
<210> 26
   <211> 4
   <212> PRT
   <213> botulinum toxin
<400> 26
   Asn Ile Ser Glu
   1
<210> 27
   <211> 4
   <212> PRT
   <213> botulinum toxin
<400> 27
   Thr Pro Gln Asp
   1
<210> 28
   <211> 4
   <212> PRT
   <213> botulinum toxin
<400> 28
   Ser Tyr Tyr Asp
   1
<210> 29
   <211> 4
   <212> PRT
   <213> botulinum toxin
<400> 29
   Ser Asp Glu Glu
   1
<210> 30
   <211> 4
   <212> PRT
   <213> botulinum toxin
<400> 30
   Ser Ala Val Glu
   1
<210> 31
   <211> 4
   <212> PRT
   <213> botulinum toxin
<400> 31
   Ser Met Asn Glu
   1
<210> 32
   <211> 4
   <212> PRT
   <213> botulinum toxin
<400> 32
   Thr Asn Ile Glu
   1
<210> 33
   <211> 4
   <212> PRT
   <213> botulinum toxin
<400> 33
   Ser Phe Thr Glu
   1
<210> 34
   <211> 4
   <212> PRT
   <213> botulinum toxin
<400> 34
   Thr Glu Phe Asp
   1
<210> 35
   <211> 6
   <212> PRT
   <213> botulinum toxin
<400> 35
   Gly Leu Tyr Gly Ala Lys
   1 5
<210> 36
   <211> 6
   <212> PRT
   <213> botulinum toxin
<400> 36
   Gly Thr Asp Leu Asn Ile
   1 5
<210> 37
   <211> 6
   <212> PRT
   <213> botulinum toxin
<400> 37
   Gly Gln Asn Ala Asn Leu
   1 5
<210> 38
   <211> 3
   <212> PRT
   <213> botulinum toxin
<400> 38
   Ser Leu Lys
   1
<210> 39
   <211> 3
   <212> PRT
   <213> botulinum toxin
<400> 39
   Ser Leu Arg
   1
<210> 40
   <211> 3
   <212> PRT
   <213> botulinum toxin
<400> 40
   Ser Phe Arg
   1
<210> 41
   <211> 3
   <212> PRT
   <213> botulinum toxin
<400> 41
   Thr Thr Lys
   1
<210> 42
   <211> 3
   <212> PRT
   <213> botulinum toxin
<400> 42
   Thr Gln Lys
   1
<210> 43
   <211> 3
   <212> PRT
   <213> botulinum toxin
<400> 43
   Thr Gly Arg
   1
<210> 44
   <211> 3
   <212> PRT
   <213> botulinum toxin
<400> 44
   Ser Val Lys
   1
<210> 45
   <211> 7
   <212> PRT
   <213> botulinum toxin
<400> 45
   Lys Asn Gly Asp Ser Ser Tyr
   1 5
<210> 46
   <211> 8
   <212> PRT
   <213> botulinum toxin
<400> 46
   Lys Asp Val Phe Glu Ala Lys Tyr
   1 5